# EUROPEAN PATENT SPECIFICATION

(11) **EP 3 551 181 B1**
(45) Date of publication and mention of the grant of the patent: **18.11.2020**
(21) Application number: 18701303.2
(22) Date of filing: 17.01.2018
(51) Int. Cl.: A61K 31/4152, A61K 47/02, A61K 9/08, A61P 25/00, A61K 9/00

(54) **TREATMENT COMPRISING ORAL OR GASTRIC ADMINISTRATION OF EDARAVONE**
BEHANDLUNG MIT ORALER ODER GASTRISCHER VERABREICHUNG VON EDARAVON
TRAITEMENT COMPRENANT L'ADMINISTRATION ORALE OU GASTRIQUE D'ÉDARAVONE

(30) Priority: 17.01.2017 EP 17151741; 06.07.2017 EP 17180087; 06.07.2017 WO PCT/EP2017/067005
(43) Date of publication of application: 16.10.2019
(60) Divisional application: 20201851.1
(73) Proprietor: Treeway TW001 B.V., 3047 AL Rotterdam (NL)
(72) Inventor: MOOLENAAR, Sytske Hyke, 3047 AL Rotterdam (NL); VAN DER GEEST, Ronald, 4835 AA Breda (NL)
(74) Representative: Nederlandsch Octrooibureau
(86) International application number: PCT/EP2018/051097
(87) International publication number: WO 2018/134243

(56) References cited:
- CN-A- 102 349 893
- ISHIZAWA M ET AL: "An antioxidant treatment potentially protects myocardial energy metabolism by regulating uncoupling protein 2 expression in a chronic @b-adrenergic stimulation rat model", LIFE SCIENCES, PERGAMON PRESS, OXFORD, GB, vol. 78, no. 25, 15 May 2006 (2006-05-15), pages 2974-2982, XP028050767, ISSN: 0024-3205, DOI: 10.1016/J.LFS.2006.02.029 [retrieved on 2006-05-15]
- MITSUBISHI TANABE PHARMA CORPORATION: "Radicut Injection 30 mg", INTERNET CITATION, 1 June 2015 (2015-06-01), pages 1-8, XP002777674, Retrieved from the Internet: URL:http://www.e-search.ne.jp/~jpr/PDF/MT1 8.PDF [retrieved on 2018-01-26]
- PARIKH ANKIT ET AL: "Development of a novel oral delivery system of edaravone for enhancing bioavailability", INTERNATIONAL JOURNAL OF PHARMACEUTICS (KIDLINGTON), vol. 515, no. 1-2, 24 October 2016 (2016-10-24), pages 490-500, XP002770642, cited in the application
- SHU-SHENG JIAO ET AL: "Edaravone alleviates Alzheimer's disease-type pathologies and cognitive deficits", PROCEEDINGS NATIONAL ACADEMY OF SCIENCES PNAS, vol. 112, no. 16, 6 April 2015 (2015-04-06), pages 5225-5230, XP055445120, US ISSN: 0027-8424, DOI: 10.1073/pnas.1422998112 & SHU-SHENG JIAO ET AL: "Edaravone alleviates Alzheimer's disease-type pathologies and cognitive deficits (Supporting Information)", PNAS, 6 April 2015 (2015-04-06), pages 1-10, XP055466417, DOI: 10.1073/pnas.1422998112

## Description

### Field of the invention

This invention relates to the use of 3-methyl-1-phenyl-2-pyrazolin-5-one (edaravone) in medical treatment of a neurodegenerative disease or a cerebrovascular disease in humans, said treatment comprising oral or gastric administration of a liquid pharmaceutical composition comprising water and edaravone.

### Background of the invention

ALS is a neurodegenerative disorder, which affects both the upper motor neurons, located in the brain, and the lower motor neurons, located in the spinal cord and brainstem. Upper motor neuron degeneration generally causes muscle spasticity, while lower motor neuron degeneration causes muscle weakness, muscle atrophy and twitching.

Early symptoms of ALS typically include muscle weakness in the hands, arms, legs or feet, causing weakness or spasticity in these body parts. The disease may also present itself in the muscles controlling speech or swallowing resulting in difficult chewing, speaking, swallowing, and breathing. As the disease progresses, it spreads to other parts of the body leading to progressive muscle weakness and paralysis. ALS patients ultimately lose their ability to initiate and control all voluntary movement and neuromuscular respiratory failure makes breathing increasingly difficult. Early symptoms and the development of the disease vary with each individual.

Sensory nerves and the autonomic nervous system remain unaffected, leaving hearing, sight, touch, smell, and taste intact as well as the involuntary muscles such as those that control heartbeat, gastrointestinal tract, bowel and bladder function. Cognitive function generally remains unaffected as well.

Most people who develop ALS are between the ages of 40 and 70, but the disease can also occur at a younger age. Prevalence has been found to increase with age. Although ALS is classified as a rare disease it is the most common motor neuron disease. About one or two out of 100,000 people develop ALS each year while the prevalence of ALS is estimated to be about two cases per 100,000 population, with increasing numbers due to the aging population.

Up till recently, Riluzole was the only approved drug for ALS. Its effect is believed to reside in its potential to reduce signaling of glutamate, a neurotransmitter that has been found to be present in higher levels in people with ALS. The drug has been found to have a limited beneficial effect on the symptoms of ALS as well on the progression of the disease. It would improve survival, but only to a modest extent.

In May 2017, the U.S. Food and Drug Administration approved Radicava (edaravone) to treat patients with amyotrophic lateral sclerosis (ALS), commonly referred to as Lou Gehrig's disease. Radicava is an intravenous infusion given by a health care professional. It is administered with an initial treatment cycle of daily dosing for 14 days, followed by a 14-day drug-free period. Subsequent treatment cycles consist of dosing on 10 of 14 days, followed by 14 days drug-free. The efficacy of edaravone for the treatment of ALS was demonstrated in a six-month clinical trial conducted in Japan. In the trial, 137 participants were randomized to receive edaravone or placebo. At Week 24, individuals receiving edaravone declined less on a clinical assessment of daily functioning compared to those receiving a placebo.

Radicut® is an aqueous solution of edaravone for the treatment of neurological disoders in humans by intravenous administration.

Edaravone is a nootropic and neuroprotective agent used to aid neurological recovery following acute brain ischemia and subsequent cerebral infarction. It acts as a potent antioxidant and strongly scavenges free radicals, protecting against oxidative stress and neuronal apoptosis.

Other medications prescribed to ALS patients are aimed at improving quality of life and relieving symptoms of ALS such as muscle cramps and spasms, constipation, fatigue, excessive salivation and phlegm, pain, depression, and sleep problems.

EP-A 1 405 637 describes the use of edaravone in the treatment of motor neuron diseases, including ALS.

EP-A 1 714 960 concerns the use of edaravone in the treatment of ALS with one or more drug holiday periods during the period of treatment.

EP-A 2 754 440 describes the use of edaravone for treating ALS in specific patient populations, wherein the agent is administered by repeating a 14-day administration period and a 14-day drug holiday period, or by establishing an initial 14-day administration period and an initial 14-day drug holiday period and then repeating an administration period for 10 out of 14 days and a 14-day drug holiday period.

WO 2012/019381 describes an oral pharmaceutical composition containing edaravone and cyclodextrin in a weight ratio of edaravone to cyclodextrin of 1:6-100. The preparation method comprises the following steps:
- mixing β- cyclodextrin or the mixture of cyclodextrins containing β- cyclodextrin with 1-5 times of water in weight,
- adding edaravone or its solution in organic solvent into the cyclodextrin solution,
- grinding or stirring, and
- evaporating water at the temperature no higher than 60°C, drying by decompression.

It is well recognized in the prior art that the oral bioavailability of edaravone is low and investigations have been carried out in order to provide oral formulations of edaravone that achieve improved oral bioavailability.

Rong et al. (Hydroxypropyl-Sulfobutyl-β-Cyclodextrin Improves the Oral Bioavailability of Edaravone by Modulating Drug Efflux Pump of Enterocytes, Journal of Pharmaceutical Sciences (2013), DOI 10.1002/jps.23807, 1-13) describe a study in which the effect of hydroxypropyl-sulfobutyl-β-cyclodextrin on the bioavailability and intestinal absorption of edaravone was investigated. It was found that the inclusion complex of edaravone-cyclodextrin improved the water solubility of edaravone and enhanced the bioavailability of bioavailability of edaravone in rats. Table 2 of this article shows that the absolute bioavailability (F_{abs}) of orally administered 'raw' edaravone (suspended with 0.5% CMC-Na) was only 5.23% (compared to 100% bioavailability of intravenously administered edaravone). Table 2 further shows that the oral bioavailability of edaravone could be improved by more than factor 10 by complexing edaravone with cyclodextrin.

Parikh et al. (Development of a novel oral delivery system of edaravone for enhancing bioavailability, International Journal of Pharmaceutics 515 (2016) 490-500) discuss the development of an oral delivery system of edaravone. The authors describe a Novel Oral Delivery System of edaravone (NODS) that is made up of a mixture of Labrasol and an acidic aqueous system that was optimized on the basis of a solubility and stability study. The NODS delivery system contained 30 mg/mL of edaravone. The *in-vivo* oral bioavailability of the NODS delivery system was investigated in adult rats using an equivalent dose of 30 mg/kg edaravone. It was found that the oral bioavailability of the NODS delivery system was 5.7 times higher than that of an edaravone suspension containing 30 mg/mL edaravone and 0.5% sodium carboxymethyl cellulose (see Table 2).

Parikh et al. (Lipid-based nanosystem of edaravone: development, optimization, characterization and in vitro/in vivo evaluation, Drug Delivery 24(1); (2017), 962-978) describe a study that aimed at enabling oral use of edaravone by developing a lipid-based nanosystem (LNS). The components of LNS including oil, surfactants, and co-surfactants were selected based on their potential to maximize the solubilization in gastrointestinal (GI) fluids, reduce its glucuronidation and improve transmembrane permeability. A liquid LNS (L-LNS) in the form of a micro-emulsion was prepared, comprising Capryol™ PGMC (Oil), Cremophor® RH 40:Labrasol® :TPGS 1000 (1:0.8:0.2) (Surfactant) and Transcutol® P (Co-surfactant). This micro-emulsion had the following characteristics: droplet size (16.25 nm), polydispersity index (0.039), %Transmittance (99.85%), and self-emulsification time (32 s). It was found that the oral bioavailability of the L-LNS was almost 11 times higher than that of an edaravone suspension containing 30 mg/mL edaravone and 0.5% sodium carboxymethyl cellulose (see Table 3).

WO 2012/019381 describes an oral pharmaceutical composition containing edaravone and cyclodextrin in a weight ratio of edaravone to cyclodextrin of 1:6-100. The preparation method comprises the following steps:
- mixing β- cyclodextrin or the mixture of cyclodextrins containing β- cyclodextrin with 1-5 times of water in weight,
- adding edaravone or its solution in organic solvent into the cyclodextrin solution,
- grinding or stirring, and
- evaporating water at the temperature no higher than 60°C, drying by decompression.

CN 101 953 832 describes an oral pharmaceutical composition comprising cyclodextrin in combination with edaravone. The examples of the Chinese patent application describe tablets, capsules and granules containing cyclodextrin-edaravone complex.

CN 105 816 423 describes various lipid-based drug delivery systems comprising edaravone. The examples of this Chinese patent application describe oral administration of edaravone (30 mg/kg) to rats using a self-micro-emulsifying drug delivery systems (SMEDDS).

The U.S. Food and Drug Administration has approved an intravenous edaravone infusion (Radicava™) to treat patients with amyotrophic lateral sclerosis (ALS). Radicava is to be administered by a health care professional. It is administered with an initial treatment cycle of daily dosing for 14 days, followed by a 14-day drug-free period. Subsequent treatment cycles consist of dosing on 10 of 14 days, followed by 14 days drug-free

Intravenous administration is a less attractive route of administration as it requires the presence of a medical practitioner and therefore does not allow self-administration. Furthermore, many patients do not like to receive a drug by injection.

### Summary of the invention

Amongst the various ways of drug delivery, oral delivery is still the most attractive and acceptable route of administration for active pharmaceutical ingredients. The oral route is preferred because of its convenience, resulting in high levels of patient acceptance and long term compliance, which in turn increases the therapeutic value of the drug. In most instances, it allows the patient to self-administer the drug without the help of a medical practitioner.

It is therefore desirable to provide edaravone dosage forms that can be administered by the oral route. Traditional oral dosage forms such as tablets and capsules, however, pose problems to patients who have difficulty in swallowing. This is often the case with patients suffering from neurodegenerative diseases, e.g. ALS patients.

The inventors have unexpectedly discovered that aqueous solutions of edaravone have a much higher oral bioavailability than was heretofore assumed. Whereas the aforementioned article by Rong et al. reported an absolute bioavailability of orally administered 'raw' edaravone of only 5.23%, the inventors have observed an absolute bioavailability of about 80% when the aqueous edaravone solution of the present invention is orally administered to human subjects. As a matter of fact, oral bioavailability of aqueous solutions containing 0.2-9 mg/mL edaravone is so high that meaningful medical effects are achieved when the aqueous solution is orally administered in a daily dose providing 30 mg to 300 mg edaravone.

Surprisingly, the aqueous edaravone solutions of the present invention achieve a high oral bioavailability without the use of edaravone in complexed form and without enhancing the solubility of edaravone by incorporating micelles or a dispersed lipid phase (nano- or micro-emulsion).

Consequently, the present invention relates to aqueous edaravone solutions for use in medical treatment of a neurodegenerative disease or a cerebrovascular disease in humans, said treatment comprising oral or gastric administration of 10-250 mL of a monophasic aqueous solution of non-complexed edaravone to provide 30-300 mg edaravone, said monophasic aqueous solution comprising at least 75 wt.% water and 0.2-9 mg/mL of edaravone according to claim 1. Radicava™ is an example of an aqueous edaravone solution that may be used in these oral or gastric treatments in diluted or non-diluted form.

### Detailed description of the invention

Accordingly, the present invention relates to a liquid pharmaceutical composition for use in medical treatment of a neurodegenerative disease or a cerebrovascular disease in humans, said liquid pharmaceutical composition being a monophasic aqueous solution of non-complexed 3-methyl-1-phenyl-2-pyrazolin-5-one (edaravone) and comprising at least 75 wt.% water and 0.2-9 mg/mL of edaravone, wherein the treatment comprises oral or gastric administration of 10-250 mL of the liquid pharmaceutical composition to provide 30-300 mg edaravone, and 0.4-4 mg edaravone per kg of body weight per day.

The term "edaravone" as used herein refers to the substance 3-methyl-1-phenyl-2-pyrazolin-5-one.

The terminology "aqueous solution of edaravone" as used herein refers to a homogenous mixture in which the edaravone is fully dissolved in water.

The term "monophasic" as used herein in relation to the aforementioned aqueous solution, refers to a liquid composition that does not contain two or more distinctive phases. Consequently, the liquid pharmaceutical composition of the present invention is not an emulsion (e.g. a micro-emulsion, a nano-emulsion or a micellar suspension/solution).

The term "non-complexed edaravone" as used herein, means that the edaravone is not present in the liquid pharmaceutical composition in the form of a complex with an complexing agent, such as beta-cyclodextrin.

The term "treatment" as used herein encompasses both therapeutic and palliative treatment. The term "gastric administration" refers to administration into the stomach via a tube through the nasal passage (NG tube) or a tube in the abdomen (PEG tube).

The liquid pharmaceutical composition of the present invention is preferably used for treatment of mammals, more preferably for treatment of humans.

The treatment according to the present invention preferably comprises orally or gastrically administering the liquid pharmaceutical composition to the patient at least once daily during a period of at least 2 weeks, more preferably during a period of at least 4 weeks.

The liquid pharmaceutical composition is preferably administered orally or gastrically in an amount providing 50-200 mg edaravone, more preferably in an amount providing 80-160 mg edaravone.

The present invention also encompasses treatments in which two or more doses of the liquid pharmaceutical composition are administered per day. Preferably, the total amount of edaravone that is administered orally or gastrically does not exceed 300 mg edaravone per day, more preferably it does not exceed 200 mg edaravone per day, most preferably it does not exceed 160 mg edaravone per day.

In some cases it may be advisable to adjust the edaravone dosage to the bodyweight of the patient. Typically, the liquid pharmaceutical composition of the present invention is administered in an amount providing 0.4-4 mg edaravone per kg of bodyweight per day, more preferably in an amount providing 0.8-3.7 mg edaravone per kg of bodyweight per day, most preferably in an amount providing 1-3.5 mg edaravone per kg of bodyweight per day.

In accordance with one preferred embodiment of the present invention the liquid pharmaceutical composition is administered as a dilute edaravone solution in an amount of 40-250 mL, more preferably in an amount of 80-200 mL, most preferably in an amount of 100-150 mL. Typically, this dilute edaravone solution contains 0.3-1 mg/mL edaravone, more preferably, 0.4-0.8 mg/mL edaravone. The dilute edaravone solution may suitably be prepared shortly before oral or gastric administration by diluting a concentrated edaravone solution with aqueous liquid or by mixing a dry edaravone formulation with aqueous liquid.

In another preferred embodiment, the liquid pharmaceutical composition is administered in the form of a concentrated edaravone solution in amount of 20-150 mL, more preferably of 30-100 mL. The concentrated edaravone solution typically contains 1-9 mg/mL edaravone, more preferably 1.1-7 mg/mL edaravone, even more preferably 1.15-4 mg/mL edaravone, most preferably 1.2-2 mg/mL edaravone.

In yet another preferred embodiment of the present treatment the patient has fasted for at least 1 hour before oral or gastric administration of the liquid pharmaceutical composition.

The water content of the liquid pharmaceutical composition preferably is at least 85 wt.%.

Besides edaravone and water, the liquid pharmaceutical composition preferably contains one or more further components. Examples of such additional components include antioxidants, pH-regulators, preservatives and sodium chloride.

In accordance with one embodiment of the present invention, the liquid pharmaceutical composition has been manufactured as a ready-to-use liquid composition. According to a preferred embodiment, this ready-to-use liquid pharmaceutical composition contains alkali metal bisulfite and edaravone in a molar ratio of 1:2 to 2:1, more preferably in a molar ratio of 2:3 to 3:2, said alkali metal bisulfite being selected from sodium bisulfite, potassium bisulfite and combinations thereof. Most preferably, the alkali metal bisulfite employed in the liquid pharmaceutical composition is sodium bisulfite.

According to a further preferred embodiment, the ready-to-use liquid composition contains L-cysteine and edaravone in a weight ratio of 1:5 to 1:1, more preferably in a weight ratio of 1:4 to 1:2.

The pH of the ready-to-use liquid composition is typically in the range of 3.0 to 9.0, more preferably in the range of 3.0-6.0. In case the ready-to-use liquid composition is a concentrated solution, as described herein before, the pH is preferably in the range of 3.0 to 4.5. In case the ready-to-use liquid composition is a dilute solution, as described herein before, the pH of the composition is preferably in the range of 3.5 to 6.0.

The ready-to-use liquid composition in the form of the concentrated solution preferably has an osmolarity in the range of 250-320 mOsm/L.

In another preferred embodiment, the ready-to-use liquid composition does not comprise a nonionic surfactant. Even more preferably, the ready-to-use liquid composition does not contain a surfactant.

The ready-to-use liquid composition preferably is a sterile solution.

In another embodiment of the present invention, the liquid pharmaceutical composition is freshly prepared prior to oral or gastric administration by mixing a dry particulate edaravone formulation comprising edaravone with aqueous liquid.

The inventors have unexpectedly discovered that the rate at which edaravone dissolves in water is increased substantially in the presence of an alkalizing agent. Accordingly, the liquid pharmaceutical composition is preferably prepared prior to oral or gastric administration by mixing a dry particulate pharmaceutical composition containing edaravone and alkalizing agent with aqueous liquid.

Preferably, the freshly prepared liquid composition contains 0.3-9 mg/mL, more preferably 0.5-4 mg/mL and most preferably 0.8-2 mg/mL of water-soluble alkalizing agent. The water-soluble alkalizing agent is preferably selected from oxides and hydroxides of alkaline metals; oxides and hydroxides of alkali-earth metals; Al(OH)₃; Fe₂O₃; salts of weak organic and weak inorganic acids, alkaline amines; alkaline amino acids; and combinations thereof.

The oxides and hydroxides of alkaline metals are preferably selected from NaOH, KOH, LiOH and combinations thereof. The oxides and hydroxides of alkali-earth metals are preferably selected from Ca(OH)₂, CaO, Mg(OH)₂ MgO and combinations thereof. The salts of weak organic and weak inorganic acids are preferably selected from carbonate, bicarbonate, borate, carboxylate (e.g. lactate, citrate, acetate, formate and oxalate), phosphate, sulfate and combinations thereof. The alkaline amines are preferably selected from tris(hydroxymethyl)aminomethane, ethanolamine, diethanolamine, triethanolamine, N-methyl-glucamine, glucosamine, ethylenediamine, diethylamine, triethylamine, isopropylamine, diisopropylamine, ammonia and combinations thereof. The alkaline amino acids are preferably selected from arginine, histidine, lysine and combinations thereof. The invention encompasses the use of the above mentioned water-soluble alkalizing agents in the form of pharmaceutically acceptable salts and hydrates.

According to a particularly preferred embodiment, the water-soluble alkalizing agent is selected from tris(hydroxymethyl)aminomethane, phosphates (e.g. Na₃PO₄) and combinations thereof.

The freshly prepared liquid composition preferably has a pH of at least 6.0, more preferably of at least 6.5, more preferably of at least 6.8. The pH of the freshly prepared liquid composition typically does not exceed 9.0, more preferably does not exceed 8.8 and most preferably does not exceed 8.5.

According to another preferred embodiment, the freshly prepared liquid composition contains 5-40 mg/mL, more preferably 6-25 mg/mL polyol selected from mannitol, sorbitol, xylitol, maltitol, lactitol and combinations thereof.

The freshly prepared liquid composition preferably contains 0.03-30 mg/mL, more preferably 0.05-20 mg/mL surfactant.

According to a particularly preferred embodiment, the freshly prepared liquid composition contains at least 0.03 mg/mL, more preferably at least 0.05 mg/mL nonionic surfactant. The nonionic surfactant is preferably selected from Poloxamers, polysorbates and combinations thereof. Poloxamer is a nonionic triblock copolymers composed of a central hydrophobic chain of polyoxypropylene flanked by two hydrophilic chains of polyoxyethylene.

According to a preferred embodiment, the liquid pharmaceutical composition contains less than 3 wt.%, preferably less than 1 wt.% of water-soluble organic solvent selected from polyethylene glycol (e.g. PEG200-10,000), propylene glycol, glycerol, diethylene glycol monoethyl ether (e.g Transcutol HP, Transcuto lP), polyoxyl castor oils (e.g. Cremophor RH 40, Cremophor EL), polyoxylglycerides (e.g. Labrasol), polyoxyethylene sorbitan fatty acid esters (e.g. Tween 20, Tween 80), water-soluble forms of vitamin E (e.g. TPGS 1000) and ethanol.

The liquid pharmaceutical composition preferably contains not more than 5 wt.%, more preferably not more than 3 wt.% and even more preferably not more than 1 wt.% organic substances other than edaravone.

The liquid pharmaceutical composition of the present invention is preferably used to treat a neurodegenerative disease; cerebral amyloid angiopathy (CAA); an auto-immune disease; myocardial infarction or a cerebrovascular disease. More preferably, the liquid pharmaceutical composition is used to treat a neurodegenerative disease or a cerebrovascular disease.

The liquid pharmaceutical composition is used to treat a neurodegenerative disease, more preferably a neurodegenerative disease selected from amyotrophic lateral sclerosis (ALS) and Alzheimer's disease. Most preferably, the liquid pharmaceutical composition is used to treat ALS.

According to a particularly preferred embodiment, the present treatment of a disease comprises palliative treatment.

The invention is further illustrated by the following examples.

### Examples

### Example 1

A study was conducted in which the edaravone solution contained in Radicut® ampoules was both orally and intravenously administered to dogs to provide a single dosage of 60 mg edaravone.

The study was conducted in a group of 4 male beagle dogs. Animals were given a single oral administration of the edaravone solution contained in two Radicut® ampoules (each ampoule containing 30 mg edaravone /20 ml solution). After a wash-out period of 2 days, the animals received an intravenous infusion of two Radicut® ampoules (15 minutes infusion time).

Blood samples were taken just before and at regular intervals after administration, and the edaravone plasma concentration of each sample was determined. The averaged results of these measurements are shown in Table 1. Parameters indicative of the relative bioavailability are presented in Table 2.

**Table 1**

| **Time (hrs)** | **Edaravone plasma concentration (ng/ml)** | |
|---|---|---|
| | **oral administration** | **i.v. infusion** |
| Pre | 0 | 0 |
| 0.083 | 2307 | 3060 |
| 0.17 | 994 | n.d. |
| 0.25 | 491 | 4383 |
| 0.5 | 185 | 648 |
| 1 | 84 | 140 |
| 1.5 | 51 | n.d. |
| 2 | 36 | 49 |
| 4 | 11 | 17 |
| 6 | 5 | n.d. |
| 8 | n.d. | 6 |
| 10 | n.d. | 5 |

| | | |
|---|---|---|
| n.d.: not determined | | |

**Table 2**

| | **oral administration** | **i.v. infusion** |
|---|---|---|
| Tₘₐₓ (min) | 5 | 15 |
| Cₘₐₓ (ng/ml) | 2310 | 4380 |
| AUCₗₐₛₜ (h.ng/ml)¹ | 542 | 1600 |
| AUC_{0-inf} (h.ng/ml)² | 553 | 1630 |

| | | |
|---|---|---|
| AUCₗₐₛₜ is the area under the plasma concentration-time curve from time of administration until the last measurable plasma concentration ² AUC_{0-inf} is the area under the plasma concentration-time curve from time of administration until (extrapolated) infinity | | |

These results demonstrate that the orally administered edaravone exhibits rapid absorption with peak maximum concentrations reached at about 5 min after administration and shows good systemic bioavailability (F_{abs} = 34%) compared to iv infusion.

### Example 2

A single dose, randomized, two-period, cross-over study was conducted in 18 healthy male and female human subjects.

The subjects received:
• 140 mg edaravone (p.o) in the form of 100 mL of a freshly prepared solution containing 1.5 grams of the dry formulation shown in Table 3
• 60 mg edaravone (i.v. 60 minutes), using two Radicut® ampoules (30 mg edaravone in 20 mL solution)

**Table 3**

| | **wt.%** |
|---|---|
| Edaravone (micronized) | 9.3 |
| Mannitol | 56.8 |
| Sodium orthophosphate | 33.3 |
| Sodium lauryl sulfate | 0.5 |
| Total | 100.0 |

Blood samples were taken just before and at regular intervals after administration, and the edaravone plasma concentration of each sample was determined. The averaged results of these measurements are shown in Table 4. Parameters indicative of the relative bioavailability are presented in Table 5.

**Table 4**

| **Time (hrs)** | **Edaravone plasma concentration (ng/ml)** | |
|---|---|---|
| | **oral administration (140 mg)** | **i.v. infusion (60 mg)** |
| Pre | 0 | 0 |
| 0.083 | 1197 | n.d. |
| 0.17 | 2358 | 380 |
| 0.25 | 2627 | n.d. |
| 0.5 | 1806 | 713 |
| 1 | 689 | 990 |
| 1.5 | 354 | 321 |
| 2 | 203 | n.d. |
| 4 | 68 | 42 |
| 6 | 24 | 14 |
| 8 | 13 | 8 |
| 10 | 9 | 5 |

| | | |
|---|---|---|
| n.d.: not determined | | |

**Table 5**

| | **oral administration** | **i.v. infusion** |
|---|---|---|
| Tₘₐₓ (hr) | 0.25 | 1 |
| Cₘₐₓ (ng/ml) | 2936 | 1030 |
| AUCₗₐₛₜ (h.ng/ml)¹ | 2389 | 1292 |
| AUC_{0-inf} (h.ng/ml)² | 2413 | 1304 |

| | | |
|---|---|---|
| ¹ AUCₗₐₛₜ is the area under the plasma concentration-time curve from time of administration until the last measurable plasma concentration ² AUC_{0-inf} is the area under the plasma concentration-time curve from time of administration until (extrapolated) infinity | | |

These results demonstrate that the orally administered edaravone exhibits rapid absorption with peak maximum concentrations reached at about 15 min after administration and shows good systemic bioavailability (F_{abs} = 79%) compared to i.v. infusion.

## Claims

1. A liquid pharmaceutical composition for use in medical treatment of a neurodegenerative disease or a cerebrovascular disease in humans, said liquid pharmaceutical composition being a monophasic aqueous solution of non-complexed 3-methyl-1-phenyl-2-pyrazolin-5-one (edaravone) and comprising at least 75 wt.% water and 0.2-9 mg/mL of edaravone, wherein the treatment comprises oral or gastric administration of 10-250 mL of the liquid pharmaceutical composition to provide 30-300 mg edaravone, and 0.4-4 mg edaravone per kg of bodyweight per day.

2. Liquid pharmaceutical composition for use in the treatment according to claim 1, wherein the liquid pharmaceutical composition is administered in an amount providing 50-200 mg edaravone.

3. Liquid pharmaceutical composition for use in the treatment according to claim 1 or 2, wherein the liquid pharmaceutical composition is administered in an amount providing not more than 300 mg edaravone per day.

4. Liquid pharmaceutical composition for use in the treatment according to any one of the preceding claims, wherein the liquid pharmaceutical composition is administered in an amount providing 0.8-3.7 mg edaravone per kg of bodyweight per day.

5. Liquid pharmaceutical composition for use in the treatment according to any one of the preceding claims, wherein the liquid pharmaceutical composition is a dilute edaravone solution containing 0.3-1 mg/mL edaravone, said dilute edaravone solution being administered in an amount of 40-250 mL.

6. Liquid pharmaceutical composition for use in the treatment according to any one of claims 1-4, wherein the liquid pharmaceutical composition is a concentrated edaravone solution containing 1-9 mg/mL edaravone, said concentrated edaravone solution being administered in an amount of 20-150 mL.

7. Liquid pharmaceutical composition for use in the treatment according to any one of the preceding claims, wherein the liquid pharmaceutical composition contains at least 85 wt.% water.

8. Liquid pharmaceutical composition for use in the treatment according to any one of the preceding claims, wherein the liquid pharmaceutical composition contains alkali metal bisulfite and edaravone in a molar ratio of 1:2 to 2:1, said alkali metal bisulfite being selected from sodium bisulfite, potassium bisulfite and combinations thereof.

9. Liquid pharmaceutical composition for use in the treatment according to any one of the preceding claims, wherein the liquid pharmaceutical composition contains L-cysteine and edaravone in a weight ratio of 1:5 to 1:1.

10. Liquid pharmaceutical composition for use in the treatment according to any one of the preceding claims, wherein the liquid pharmaceutical composition contains no non-ionic surfactant.

11. Liquid pharmaceutical composition for use in the treatment according to any one of claims 1-7, wherein the liquid pharmaceutical composition is prepared prior to oral or gastric administration by mixing a dry particulate edaravone formulation comprising edaravone with aqueous liquid.

12. Liquid pharmaceutical composition for use in the treatment according to claim 11, wherein the dry particulate edaravone formulation comprises an alkalizing agent.

13. Liquid pharmaceutical composition for use in the treatment according to claim 12, wherein the alkalizing agent is selected from oxides and hydroxides of alkaline metals; oxides and hydroxides of alkali-earth metals; Al(OH)₃; Fe₂O₃; salts of weak organic and weak inorganic acids, alkaline amines; alkaline amino acids; and combinations thereof.

14. Liquid pharmaceutical composition for use in the treatment according to any one of claims 11-13, wherein the liquid pharmaceutical composition has a pH in the range of 6.0 to 9.0.

15. Liquid pharmaceutical composition for use in the treatment according to any one of the preceding claims, wherein the liquid pharmaceutical composition is used to treat a neurodegenerative disease selected from amyotrophic lateral sclerosis (ALS) and Alzheimer's disease.

16. Liquid pharmaceutical composition for use in the treatment according to any one of the preceding claims, wherein the treatment comprises orally or gastrically administering the liquid pharmaceutical composition at least once daily during a period of at least 2 weeks.

## Patentansprüche

1. Flüssige pharmazeutische Zusammensetzung zur Verwendung bei der medizinischen Behandlung einer neurodegenerativen Krankheit oder einer zerebrovaskulären Krankheit beim Menschen, wobei die flüssige pharmazeutische Zusammensetzung eine einphasige wässrige Lösung von unkomplexiertem 3-Methyl-1-phenyl-2-pyrazolin-5-on (Edaravon) ist und wenigstens 75 Gew.-% Wasser und 0,2-9 mg/ml Edaravon umfasst, wobei die Behandlung die orale oder gastrische Verabreichung von 10 bis 250 ml der flüssigen pharmazeutischen Zusammensetzung umfasst, wobei man 30 bis 300 mg Edaravon, und 0,4 bis 4 mg Edaravon pro kg Körpergewicht pro Tag erhält.

2. Flüssige pharmazeutische Zusammensetzung zur Verwendung bei der Behandlung gemäß Anspruch 1, wobei die flüssige pharmazeutische Zusammensetzung in einer Menge verabreicht wird, die 50 bis 200 mg Edaravon ergibt.

3. Flüssige pharmazeutische Zusammensetzung zur Verwendung bei der Behandlung gemäß Anspruch 1 oder 2, wobei die flüssige pharmazeutische Zusammensetzung in einer Menge verabreicht wird, die nicht mehr als 300 mg Edaravon pro Tag ergibt.

4. Flüssige pharmazeutische Zusammensetzung zur Verwendung bei der Behandlung gemäß einem der vorstehenden Ansprüche, wobei die flüssige pharmazeutische Zusammensetzung in einer Menge verabreicht wird, die 0,8 bis 3,7 mg Edaravon pro kg Körpergewicht pro Tag ergibt.

5. Flüssige pharmazeutische Zusammensetzung zur Verwendung bei der Behandlung gemäß einem der vorstehenden Ansprüche, wobei die flüssige pharmazeutische Zusammensetzung eine verdünnte Edaravonlösung ist, die 0,3 bis 1 mg/ml Edaravon enthält, wobei die verdünnte Edaravonlösung in einer Menge von 40 bis 250 ml verabreicht wird.

6. Flüssige pharmazeutische Zusammensetzung zur Verwendung bei der Behandlung gemäß einem der Ansprüche 1 bis 4, wobei die flüssige pharmazeutische Zusammensetzung eine konzentrierte Edaravonlösung ist, die 1 bis 9 mg/ml Edaravon enthält, wobei die konzentrierte Edaravonlösung in einer Menge von 20 bis 150 ml verabreicht wird.

7. Flüssige pharmazeutische Zusammensetzung zur Verwendung bei der Behandlung gemäß einem der vorstehenden Ansprüche, wobei die flüssige pharmazeutische Zusammensetzung wenigstens 85 Gew.-% Wasser enthält.

8. Flüssige pharmazeutische Zusammensetzung zur Verwendung bei der Behandlung gemäß einem der vorstehenden Ansprüche, wobei die flüssige pharmazeutische Zusammensetzung Alkalimetallhydrogensulfit und Edaravon in einem Stoffmengenverhältnis von 1:2 bis 2:1 enthält, wobei das Alkalimetallhydrogensulfit aus Natriumhydrogensulfit, Kaliumhydrogensulfit und Kombinationen davon ausgewählt ist.

9. Flüssige pharmazeutische Zusammensetzung zur Verwendung bei der Behandlung gemäß einem der vorstehenden Ansprüche, wobei die flüssige pharmazeutische Zusammensetzung L-Cystein und Edaravon in einem Gewichtsmengenverhältnis von 1:5 bis 1:1 enthält.

10. Flüssige pharmazeutische Zusammensetzung zur Verwendung bei der Behandlung gemäß einem der vorstehenden Ansprüche, wobei die flüssige pharmazeutische Zusammensetzung kein nichtionisches Tensid enthält.

11. Flüssige pharmazeutische Zusammensetzung zur Verwendung bei der Behandlung gemäß einem der Ansprüche 1 bis 7, wobei die flüssige pharmazeutische Zusammensetzung vor der oralen oder gastrischen Verabreichung vorbereitet wird, indem man eine trockene teilchenförmige Edaravonzubereitung, die Edavaron umfasst, mit wässriger Flüssigkeit mischt.

12. Flüssige pharmazeutische Zusammensetzung zur Verwendung bei der Behandlung gemäß Anspruch 11, wobei die trockene teilchenförmige Edaravonzubereitung ein Alkalisierungsmittel umfasst.

13. Flüssige pharmazeutische Zusammensetzung zur Verwendung bei der Behandlung gemäß Anspruch 12, wobei das Alkalisierungsmittel aus Oxiden und Hydroxiden von Alkalimetallen, Oxiden und Hydroxiden von Erdalkalimetallen, Al(OH)₃, Fe₂O₃, Salzen von schwachen organischen und schwachen anorganischen Säuren, alkalischen Aminen, alkalischen Aminosäuren und Kombinationen davon ausgewählt ist.

14. Flüssige pharmazeutische Zusammensetzung zur Verwendung bei der Behandlung gemäß einem der Ansprüche 11 bis 13, wobei die flüssige pharmazeutische Zusammensetzung einen pH-Wert im Bereich von 6,0 bis 9,0 aufweist.

15. Flüssige pharmazeutische Zusammensetzung zur Verwendung bei der Behandlung gemäß einem der vorstehenden Ansprüche, wobei die flüssige pharmazeutische Zusammensetzung verwendet wird, um eine neurodegenerative Krankheit, die aus amyotropher Lateralsklerose (ALS) und Alzheimer-Krankheit ausgewählt ist, zu behandeln.

16. Flüssige pharmazeutische Zusammensetzung zur Verwendung bei der Behandlung gemäß einem der vorstehenden Ansprüche, wobei die Behandlung die orale oder gastrische Verabreichung der flüssigen pharmazeutischen Zusammensetzung wenigstens einmal täglich während eines Zeitraums von wenigstens 2 Wochen umfasst.

## Revendications

1. Composition pharmaceutique liquide destinée à être utilisée dans un traitement médical d'une maladie neurodégénérative ou d'une maladie cérébrovasculaire chez des êtres humains, ladite composition pharmaceutique liquide étant une solution aqueuse monophasique de 3-méthyl-1-phényl-2-pyrazoline-5-one non complexé (édaravone) et comprenant au moins 75 % en poids d'eau et 0,2 à 9 g/ml d'édaravone, dans laquelle le traitement comprend une administration orale ou gastrique de 10 à 250 ml de la composition pharmaceutique liquide pour fournir 30 à 300 mg d'édaravone, et 0,4 à 4 mg d'édaravone par kg de poids corporel par jour.

2. Composition pharmaceutique liquide à utiliser dans le traitement selon la revendication 1, dans laquelle la composition pharmaceutique liquide est administrée selon une quantité fournissant 50 à 200 mg d'édaravone.

3. Composition pharmaceutique liquide à utiliser dans le traitement selon la revendication 1 ou 2, dans laquelle la composition pharmaceutique liquide est administrée selon une quantité ne fournissant pas plus de 300 mg d'édaravone par jour.

4. Composition pharmaceutique liquide à utiliser dans le traitement selon l'une quelconque des revendications précédentes, dans laquelle la composition pharmaceutique liquide est administrée selon une quantité fournissant 0,8 à 3,7 mg d'édaravone par kg de poids corporel par jour.

5. Composition pharmaceutique liquide à utiliser dans le traitement selon l'une quelconque des revendications précédentes, dans laquelle la composition pharmaceutique liquide est une solution d'édaravone diluée contenant 0,3 à 1 mg/ml d'édaravone, ladite solution d'édaravone diluée étant administrée selon une quantité de 40 à 250 ml.

6. Composition pharmaceutique liquide à utiliser dans le traitement selon l'une quelconque des revendications 1 à 4, dans laquelle la composition pharmaceutique liquide est une solution d'édaravone concentrée contenant 1 à 9 mg/ml d'édaravone, ladite solution d'édaravone concentrée étant administrée selon une quantité de 20 à 150 ml.

7. Composition pharmaceutique liquide à utiliser dans le traitement selon l'une quelconque des revendications précédentes, dans laquelle la composition pharmaceutique liquide contient au moins 85 % en poids d'eau.

8. Composition pharmaceutique liquide à utiliser dans le traitement selon l'une quelconque des revendications précédentes, dans laquelle la composition pharmaceutique liquide contient du bisulfite de métal alcalin et de l'édaravone selon un rapport molaire de 1:2 à 2:1, ledit bisulfite de métal alcalin étant choisi parmi du bisulfite de sodium, bisulfite de potassium et des combinaisons de ceux-ci.

9. Composition pharmaceutique liquide à utiliser dans le traitement selon l'une quelconque des revendications précédentes, dans laquelle la composition pharmaceutique liquide contient une L-cystéine et de l'édaravone selon un rapport en poids de 1:5 à 1:1.

10. Composition pharmaceutique liquide à utiliser dans le traitement selon l'une quelconque des revendications précédentes, dans laquelle la composition pharmaceutique liquide ne contient aucun tensioactif non-ionique.

11. Composition pharmaceutique liquide à utiliser dans le traitement selon l'une quelconque des revendications 1 à 7, dans laquelle la composition pharmaceutique liquide est préparée avant une administration orale ou gastrique en mélangeant une formulation d'édaravone particulaire sèche comprenant de l'édaravone avec un liquide aqueux.

12. Composition pharmaceutique liquide à utiliser dans le traitement selon la revendication 11, dans laquelle la formulation d'édaravone particulaire sèche comprend un agent alcalinisant.

13. Composition pharmaceutique liquide à utiliser dans le traitement selon la revendication 12, dans laquelle l'agent alcalinisant est choisi parmi des oxydes et hydroxydes de métaux alcalins ; oxydes et hydroxydes de métaux alcalino-terreux ; Al(OH)₃ ; Fe₂O₃ ; sels d'acides organiques faibles et inorganiques faibles, amines alcalines ; acides aminés alcalins ; et des combinaisons de ceux-ci.

14. Composition pharmaceutique liquide à utiliser dans le traitement selon l'une quelconque des revendications 11 à 13, dans laquelle la composition pharmaceutique liquide a un pH dans la plage de 6,0 à 9,0.

15. Composition pharmaceutique liquide à utiliser dans le traitement selon l'une quelconque des revendications précédentes, dans laquelle la composition pharmaceutique liquide est utilisée pour traiter une maladie neurodégénérative choisie parmi la sclérose latérale amyotrophique (SLA) et la maladie d'Alzheimer.

16. Composition pharmaceutique liquide à utiliser dans le traitement selon l'une quelconque des revendications précédentes, dans laquelle le traitement comprend une administration orale ou gastrique de la composition pharmaceutique liquide au moins une fois par jour pendant une période d'au moins 2 semaines.
